# EUROPEAN PATENT APPLICATION

(11) **EP 2 444 000 A1**
(43) Date of publication of application: **25.04.2012**
(21) Application number: 11163082.8
(22) Date of filing: 19.04.2011
(51) Int. Cl.: A61B 8/08, A61B 8/14, G01N 29/22, G01N 29/44, G01S 7/52, G01S 15/89

(54) **Ultrasound System for Adjusting Ultrasonic Beam Direction and Method for Operating Ultrasound System**

(30) Priority: 20.10.2010 KR 20100102649
(71) Applicant: Samsung Medison Co., Ltd., Nam-myun Hongchun-gun Kangwon do 250-875 (KR)
(72) Inventor: Kim, Jeong-Sik, 467-712, Gyeonggi-do (KR); Han, Song-yi, 158-094, Seoul (KR)
(74) Representative: Schmid, Wolfgang

(57) **Abstract**

Provided are an ultrasound system and a method for operating the ultrasound system, which may calculate a direction of an ultrasonic beam in real time and may adjust the ultrasonic beam based on the calculation result. The ultrasound system may include a probe to emit an ultrasonic beam to an object, a processor to measure a first reflection angle of the ultrasonic beam at a predetermined point in the object, a mapping table to map the measured first reflection angle to the predetermined point and to record mapping data, and a controller to determine an emission angle of the ultrasonic beam to the predetermined point based on the first reflection angle recorded in the mapping table, and to control the probe to emit the ultrasonic beam at the determined emission angle.

## Description

### CROSS-REFERENCE TO RELATED APPLICATION

This application claims the benefit of Korean Patent Application No. 10-2010-0102649, filed on October 20, 2010, in the Korean Intellectual Property Office, the disclosure of which is incorporated herein by reference.

### BACKGROUND

### 1. Field of the Invention

The present invention relates to an ultrasound system and a method for operating the ultrasound system, which may calculate a direction of an ultrasonic beam in real time and may adjust the ultrasonic beam based on a result of the calculation.

### 2. Description of the Related Art

An ultrasound system is an apparatus for transmitting, from the surface of a body, an ultrasound wave signal toward a predetermined structure, that is, an object such as a fetus or an internal organ, inside the body, and for visualizing a cross section of soft tissues or a blood flow using information of an ultrasound wave echo signal reflected from the tissues of the body.

This ultrasound system has advantages of a small size, a low cost, a real-time display, and a high stability without exposing patients and users to X-ray radiation and thus, the ultrasound system is widely used along with other diagnostic imaging systems such as an X-ray diagnosis equipment, a computerized tomography (CT) scanner, a magnetic resonance imaging (MRI) equipment, a nuclear medicine diagnosis equipment, and the like.

The ultrasound system transmits an ultrasonic beam toward a predetermined region in the body, and obtains and outputs an image using an ultrasonic echo signal reflected from the tissues of the body. When an angle of the ultrasonic echo signal reflected from the tissues of the body is a right angle, the obtained image is clearer. Currently, however, the ultrasound system does not apply a direction of the ultrasonic beam to obtain an image.

### SUMMARY

An aspect of the present invention provides an ultrasound system and a method for operating the ultrasound system, which may emit, to an object in a body, an ultrasonic beam using a probe, may sense an ultrasonic echo signal reflected from the object, may measure a reflection angle of the ultrasonic beam in real time, and may adjust an emission angle of the ultrasonic beam based on the reflection angle.

Another aspect of the present invention provides an ultrasound system and a method for operating the ultrasound system, which may calculate and display an emission angle between a virtual line of an ultrasonic echo signal reflected from an object at a right angle and a contour line of an output display, thereby improving an image resolution of the object.

Still another aspect of the present invention provides an ultrasound system and a method for operating the ultrasound system, which may emit, to an object, an ultrasonic beam for each line, may arrange reflection angles of the ultrasonic beam in a table, and may update an angle of the ultrasonic beam, thereby optimizing the ultrasonic beam emission.

Yet another aspect of the present invention provides an ultrasound system and a method for operating the ultrasound system, which may emit, to an object, an ultrasonic beam of a predetermined intensity among various intensities, and may arrange reflection angles of an ultrasonic echo signal larger than a predetermined amount in a table, thereby improving accuracy of table values.

According to an aspect of the present invention, there is provided an ultrasound system including a probe to emit an ultrasonic beam to an object, a processor to measure a first reflection angle of the ultrasonic beam at a predetermined point in the object, a mapping table to map the measured first reflection angle to the predetermined point and to record mapping data, and a controller to determine an emission angle of the ultrasonic beam to the predetermined point based on the first reflection angle recorded in the mapping table, and to control the probe to emit the ultrasonic beam at the determined emission angle.

According to another aspect of the present invention, there is provided a method for operating an ultrasonic system including emitting an ultrasonic beam to an object by a probe, measuring a first reflection angle of the ultrasonic beam at a predetermined point in the object by a processor, mapping the measured first reflection angle to the predetermined point and recording mapping data by a mapping table, and determining an emission angle of the ultrasonic beam to the predetermined point based on the first reflection angle recorded in the mapping table and controlling the probe to emit the ultrasonic beam at the determined emission angle, by a controller.

### EFFECT OF THE INVENTION

According to an aspect of the present invention, provided are an ultrasound system and a method for operating the ultrasound system, which may emit, to an object in a body, an ultrasonic beam using a probe, may sense an ultrasonic echo signal reflected from the object, may measure a reflection angle of the ultrasonic beam in real time, and may adjust an emission angle of the ultrasonic beam based on the reflection angle, thereby improving resolution of an ultrasound image.

According to another aspect of the present invention, provided are an ultrasound system and a method for operating the ultrasound system, which may calculate and display an emission angle between a virtual line of an ultrasonic echo signal reflected from an object in a body at a right angle and a contour line of a display outputting an ultrasound image, thereby obtaining information for improving an image resolution of the object.

According to still another aspect of the present invention, provided are an ultrasound system and a method for operating the ultrasound system, which may emit, to an object, an ultrasonic beam for each line, may arrange reflection angles of the ultrasonic beam in a table, and may update an emission angle of the ultrasonic beam to optimize the ultrasonic beam emission, thereby improving resolution of an ultrasound image.

According to yet another aspect of the present invention, provided are an ultrasound system and a method for operating the ultrasound system, which may emit, to an object, an ultrasonic beam of a predetermined intensity among various intensities, and may arrange reflection angles of an ultrasonic echo signal larger than a predetermined amount in a table to improve accuracy of table values, thereby optimizing the ultrasonic beam emission by enhancing the accuracy of the table values.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a view illustrating an example of irradiating an ultrasonic beam to an object according to an embodiment of the present invention;
FIG. 2 is a block diagram illustrating an internal structure of an ultrasound system according to an embodiment of the present invention; and
FIG. 3 is a flowchart illustrating a method for operating the ultrasound system of FIG. 2.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

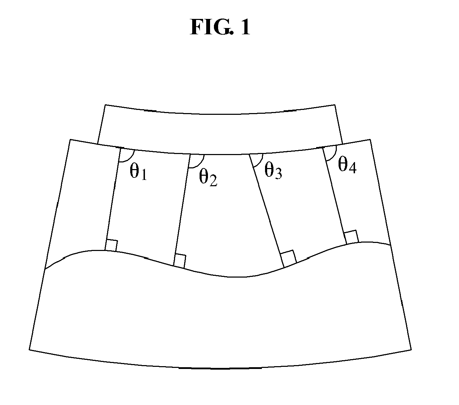

FIG. 1 is a view illustrating an example of irradiating an ultrasonic beam to an object according to an embodiment of the present invention.

The ultrasound system may emit an ultrasonic beam to an object, and may receive an ultrasonic echo signal reflected from the surface of the object at a right angle. The ultrasound system may emit an ultrasonic beam onto an object having a curved surface along the line, and may receive the ultrasonic echo signal reflected from the surface of the object at a right angle.

The ultrasound system may calculate an emission angle between a virtual line of the ultrasonic echo signal reflected from the object and a contour line. The ultrasound system may calculate an emission angle between a virtual line of the received ultrasonic echo signal reflected from the object and an arc-shaped contour line of an ultrasound image.

The ultrasound system may store, in a mapping table, reflection angles corresponding to reflections of the ultrasonic echo signal from the object. The ultrasound system may store, in a mapping table, reflection angles of the ultrasonic beam reflected from the object corresponding to the ultrasonic echo signal projected from each point of a probe.

The ultrasound system may adjust the emission angle of the ultrasonic beam based on the mapping table, and may project the ultrasonic beam. The ultrasound system may adjust the emission angle of the ultrasonic beam by referring to the mapping table storing the reflection angles of the ultrasonic echo signal reflected from the object at a right angle, and may project the ultrasonic beam to the object.

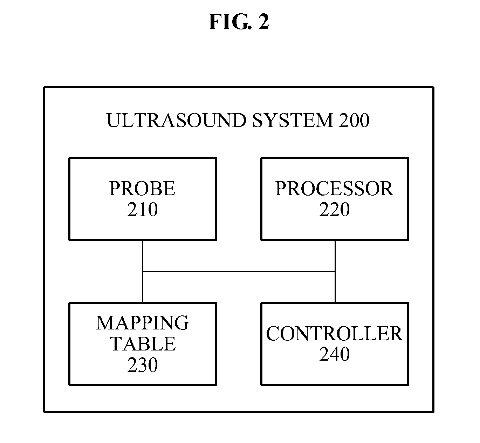

FIG. 2 is a block diagram illustrating an internal structure of an ultrasound system 200 according to an embodiment of the present invention.

The ultrasound system 200 according to an embodiment of the present invention may include a probe 210, a processor 220, a mapping table 230, and a controller 240.

The probe 210 may emit an ultrasonic beam to an object in response to an emission command of an operator using an interface. The probe 210 may emit an ultrasonic beam to an object, and may receive the ultrasonic beam reflected from the object. The probe 210 may transmit information about an emission angle and a received angle of the ultrasonic beam to the processor 220.

The processor 220 may measure a reflection angle of the ultrasonic beam at a predetermined point in the object based on information about the emission angle and the received angle of the ultrasonic beam. The processor 220 may provide the mapping table 230 with information about the predetermined point where the reflection angle was measured and information about the reflection angle measured at the predetermined point.

The mapping table 230 may map the measured reflection angle to the predetermined point and may record mapping data. When the probe 210 emits the ultrasonic beam to the object for each line and the processor 220 measures a reflection angle at a line point based on information about the emission angle of the ultrasonic beam, the mapping table 230 may arrange the reflection angles of the ultrasonic beam measured for each line in a table.

Also, when the probe 210 emits a predetermined intensity of the ultrasonic beam onto the object and the processor 220 measures reflection angles of the ultrasonic echo signal larger than a predetermined amount, the mapping table 230 may arrange the measured reflection angles of the ultrasonic echo signal in a table.

The controller 240 may determine an emission angle of the ultrasonic beam to the predetermined point based on the reflection angle recorded in the mapping table 230. The controller 240 may control the probe 210 to emit the ultrasonic beam at the determined emission angle.

The controller 240 may calculate an emission angle between a virtual line of the ultrasonic echo signal reflected from the object at a right angle and a contour line corresponding to an arc-shaped display. The controller 240 may adjust an emission angle so that a reflection angle of the ultrasonic beam emitted to the predetermined point is a right angle.

The ultrasound system 200 may process the ultrasonic beam received in the probe 210 as a signal to generate a high-resolution ultrasound image, and the ultrasonic beam may be optimized through adjustment of the emission angle by the controller 240.

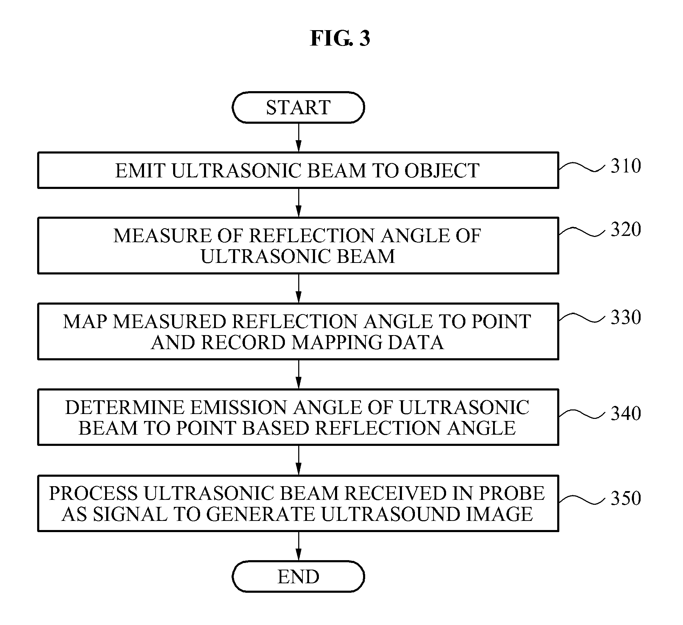

FIG. 3 is a flowchart illustrating a method for operating the ultrasound system 200 of FIG. 2.

The method for operating the ultrasound system 200 according to an embodiment of the present invention may be implemented by the ultrasound system 200 of FIG. 2. Hereinafter, the description of FIG. 3 is made with reference to FIG. 2 for ease of description.

In operation 310, the ultrasound system 200 may control the probe to emit an ultrasonic beam to an object in response to an emission command of an operator using an interface. The ultrasound system 200 may emit an ultrasonic beam to an object through the probe, and may receive an ultrasonic echo signal reflected from the object. The ultrasound system 200 may obtain information about an emission angle and a received angle of the ultrasonic beam used to measure a reflection angle of the ultrasonic beam.

In operation 320, the ultrasound system 200 may measure a reflection angle of the ultrasonic beam at a predetermined point in the object based on information about the emission angle and the received angle of the ultrasonic beam. The ultrasound system 200 may store, in the mapping table, information about the point where the reflection angle of the ultrasonic beam was measured and information about the reflection angle of the ultrasonic beam measured at the point.

In operation 330, the ultrasound system 200 may map the measured reflection angle to the point and may record mapping data in the mapping table. When the ultrasound system 200 emits the ultrasonic beam to the object for each line through the probe, the ultrasound system 200 may measure a reflection angle at a line point based on information about the emission angle of the ultrasonic beam. The ultrasound system 200 may arrange the reflection angles of the ultrasonic beam measured for each line in the mapping table.

Also, when the ultrasound system 200 emits a predetermined intensity of an ultrasonic beam to the object through the probe, the ultrasound system 200 may measure reflection angles of the ultrasonic echo signal larger than a predetermined amount. The ultrasound system 200 may arrange the measured reflection angles of the ultrasonic echo signal in the mapping table.

In operation 340, the ultrasound system 200 may determine an emission angle of the ultrasonic beam to the point based on the reflection angle recorded in the mapping table. The ultrasound system 200 may control the probe to emit the ultrasonic beam at the determined emission angle.

The ultrasound system 200 may calculate an emission angle between a virtual line of the ultrasonic echo signal reflected from the object at a right angle and a contour line corresponding to an arc-shaped display. That is, the ultrasound system 200 may adjust an emission angle so that a reflection angle of the ultrasonic beam emitted to the point is a right angle.

In operation 350, the ultrasound system 200 may perform signal processing of the ultrasonic echo signal received in the probe to generate a high-resolution ultrasound image, and the ultrasonic beam may be optimized through adjustment of the emission angle by the controller.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasound system for adjusting an ultrasonic beam direction, the system comprising:
a probe to emit an ultrasonic beam to an object;
a processor to measure a first reflection angle of the ultrasonic beam at a predetermined point in the object;
a mapping table to map the measured first reflection angle to the predetermined point
and to record mapping data; and
a controller to determine an emission angle of the ultrasonic beam to the predetermined point based on the first reflection angle recorded in the mapping table, and to control the probe to emit the ultrasonic beam at the determined emission angle.

2. The system of claim 1, wherein the controller adjusts the emission angle by the controller so that a second reflection angle of the ultrasonic beam emitted to the predetermined point is a right angle.

3. The system of claim 2, wherein the mapping table updates and records the first reflection angle based on the adjusted emission angle.

4. The system of claim 1, wherein the controller calculates the emission angle between a virtual line of the ultrasonic beam reflected from the object at a right angle and a contour line, the emission angle having the same angle as the ultrasonic beam outputted from the probe.

5. The system of claim 1, wherein the probe emits the ultrasonic beam to the object for each line, and
the mapping table arranges the first reflection angles of an ultrasonic echo signal measured for each line in a table.

6. The system of claim 5, wherein the probe emits the object with a predetermined intensity of an ultrasonic beam, and
the mapping table arranges the first reflection angles of the ultrasonic echo signal larger than a predetermined amount in a table.

7. A method for operating an ultrasonic system for adjusting an ultrasonic beam direction, the method comprising:
emitting an ultrasonic beam to an object by a probe;
measuring a first reflection angle of the ultrasonic beam at a predetermined point in the object by a processor;
mapping the measured first reflection angle to the predetermined point, and recording mapping data, by a mapping table; and
determining an emission angle of the ultrasonic beam to the predetermined point based on the first reflection angle recorded in the mapping table, and controlling the probe to emit the ultrasonic beam at the determined emission angle, by a controller.

8. The method of claim 7, further comprising:
adjusting, by the controller, the emission angle by the control so that a second reflection angle of the ultrasonic beam emitted to the predetermined point is a right angle.

9. The method of claim 8, further comprising:
updating and recording the first reflection angle based on the adjusted emission angle by the mapping table.

10. The method of claim 7, further comprising:
calculating the emission angle between a virtual line of an ultrasonic echo signal reflected from the object at a right angle and a contour line by the controller, the emission angle having the same angle as the ultrasonic beam outputted from the probe.

11. The method of claim 7, further comprising:
emitting the ultrasonic beam to the object for each line by the probe; and
arranging the first reflection angles of an ultrasonic echo signal measured for each line in a table by the mapping table.

12. The method of claim 11, further comprising:
emitting a predetermined intensity of an ultrasonic beam to the object, by the probe; and
arranging the first reflection angles of the ultrasonic beam larger than a predetermined amount in a table, by the mapping table.
